(19) Europäisches Patentamt
European Patent Office
Office européen des brevets

(11) EP 0 705 593 B1

(12) **FASCICULE DE BREVET EUROPEEN**

(45) Date de publication et mention de la délivrance du brevet:
**23.10.1996 Bulletin 1996/43**

(51) Int. Cl.[6]: **A61K 7/00**, A61K 9/127

(21) Numéro de dépôt: **95402156.4**

(22) Date de dépôt: **26.09.1995**

(54) **Composition cosmétique ou dermatologique constituée d'une émulsion huile dans eau à base de globules huileux pourvus d'un enrobage cristal liquide lamellaire**

Kosmetische und dermatologische Zusammensetzung bestehend aus einem öligen Kügelchen und mit lamellaren Flüssigkristallen beschichtete Öl in Wasser Emulsion

Dermatologic or cosmetic composition made up by an oil in water emulsion based on oily globules coated with a lamellar liquid crystal coating

(84) Etats contractants désignés:
**AT BE CH DE ES FR GB IT LI NL SE**

(30) Priorité: **07.10.1994 FR 9412005**

(43) Date de publication de la demande:
**10.04.1996 Bulletin 1996/15**

(73) Titulaire: **L'OREAL**
**F-75008 Paris (FR)**

(72) Inventeurs:
- **Ribier, Alain**
  **F-75001 Paris (FR)**
- **Simonnet, Jean-Thierry**
  **F-75011 Paris (FR)**
- **Michelet, Jacques**
  **F-91160 Champlan (FR)**

(74) Mandataire: **Lhoste, Catherine**
**L'OREAL,**
**D.P.I.,**
**90 rue du Général Roguet**
**92583 Clichy Cédex (FR)**

(56) Documents cités:
**EP-A- 0 466 236**
**FR-A- 2 633 515**
**FR-A- 2 679 788**

- **JOURNAL OF DISPERSION SCIENCE AND TECHNOLOGY, vol. 5, no. 2, 1984 pages 119-141, T. SUZUKI ET AL. 'Secondary Droplet Emulsion: Mechanism and effects of liquid crystal formation in O/W emulsion.'**
- **PARFUMS, COSMETIQUES ET AROMES., vol. 47, 1982 PARIS (FRANCE), pages 55-56, T. SUZUKI ET AL. 'Emulsions à gouttelettes secondaires.'**
- **PARFUMS, COSMETIQUES ET AROMES., vol. 47, 1982 PARIS (FRANCE), pages 55-56, T. SUZUKI ET AL. 'Emulsions à gouttelettes secondaires.'**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 705 593 B1

## Description

La présente invention concerne une composition cosmétique ou dermatologique constituée par une émulsion du type huile dans eau. Elle concerne plus particulièrement une composition constituée par une émulsion huile dans eau formée par des globules huileux pourvus d'un enrobage cristal liquide lamellaire, dispersés dans une phase aqueuse. L'invention concerne également un procédé de préparation d'une telle composition et son application au traitement de la peau et de la matière kératinique.

De nombreux composés actifs lipophiles ont un rôle important à jouer dans les domaines du soin de la peau. On peut citer comme exemples de tels actifs les vitamines lipophiles A, E ou F, les huiles essentielles, les filtres antisolaires, les alkylesters à longue chaîne d'$\alpha$-hydroxyacides, les antiinflammatoires, ainsi que les agents biostimulants des synthèses de lipides et/ou de protéines.

Leur utilisation est très répandue mais leur efficacité se voit limitée de par leur nature lipophile. Ils ne sont en effet que très partiellement absorbés par la peau et ne diffusent que très difficilement dans les assises du *stratum corneum* dans lesquelles ils sont arrêtés par la présence de compartiments aqueux au sein des espaces intercornéocytaires.

Leur introduction dans des émulsions huile dans eau stabilisées par une monocouche de tensioactifs n'améliore pratiquement pas cet état de fait étant donné que ces émulsions se cassent dès leur application sur la peau en libérant à la surface de la peau une phase huileuse contenant les actifs lipophiles qui sont très mal absorbés pour les raisons évoquées ci-dessus.

Des tentatives d'amélioration de cet état de fait ont été proposées dans l'art antérieur.

Dans JSCC 35, 45-57 (Janvier, Février 1984), JUNGINGER et al décrivent des émulsions huile dans eau dont la stabilisation est assurée par un réseau tridimentionnel cristal liquide lamellaire.

SUZUKI et al dans "Secondary droplet emulsion : Contribution of liquid crystal formation to physicochemical properties and skin moisturizing effect of cosmetic emulsion" (12ème Congrès international IFSSC, Paris septembre 1992, Abstracts, Vol I, 117-136) décrivent ces émulsions huile dans eau comme formant des superstructures ("secondary droplets"), des agrégats de gouttelettes huileuses enrobées de lamelles de cristaux liquides. Ces auteurs montrent que l'existence de ces superstructures est dépendante de la présence d'alcool gras.

Ce type d'émulsions a pour qualités principales la stabilité vis-à-vis des lâchages d'huile ainsi qu'un effet hydratant sur la peau ; il présente également des désavantages. Il est, en effet, nécessaire de mettre en oeuvre de grandes quantités de tensioactif pour constituer le réseau tridimentionnel, ce qui augmente les risques d'intolérance de la part de l'utilisateur et ce qui se traduit par un long "savonnage" (persistance d'une coloration blanche) lors de l'application sur la peau de telles compositions. De plus, la dispersion d'huile est grossière, hétérogène et l'huile est davantage sequestrée par le réseau tridimentionnel que réellement dispersée sous forme de microgouttelettes d'huile individualisées. Les gouttelettes d'huile générées par ce type d'émulsion ont une taille moyenne très supérieure aux espaces intercornéocytaires qu'elles ont à traverser et aux pores des cheveux qu'elles ont à investir, ce qui contribue à expliquer la pénétration très partielle, dans la peau et le cheveu, de la phase grasse et des actifs qui y sont dissous.

On connait encore l'article de DAHMS dans Cosmetics and Toiletries, Vol 101 Novembre 1986, qui décrit des émulsions présentant les mêmes caractéristiques et, donc, les mêmes inconvénients.

On constate par conséquent que subsistait dans l'art antérieur le besoin d'émulsions permettant une pénétration améliorée des compositions cosmétiques ou dermatologiques dans la peau et dans le cheveu.

La demanderesse a maintenant découvert une nouvelle émulsion permettant d'atteindre cet objectif.

La présente demande a donc pour objet une composition cosmétique ou dermatologique constituée par une émulsion du type huile dans eau formée de globules huileux pourvus chacun d'un enrobage cristal liquide lamellaire et dispersés dans une phase aqueuse, caractérisée en ce que chaque globule huileux contenant au moins un composé actif lipophile cosmétique ou dermatologique est unitairement enrobé d'une couche monolamellaire ou oligolamellaire obtenue à partir d'au moins un agent tensioactif lipophile, d'au moins un agent tensioactif hydrophile et d'au moins un lipide amphiphile ionique conférant à l'émulsion un pH allant de 5,5 à 7,5, les globules huileux enrobés ayant un diamètre moyen inférieur à 500 nanomètres.

La présente invention a aussi pour objet une émulsion du type huile dans eau formée de globules huileux pourvus chacun d'un enrobage cristal liquide lamellaire et dispersés dans une phase aqueuse, caractérisée en ce que chaque globule huileux est unitairement enrobé d'une couche monolamellaire ou oligolamellaire obtenue à partir d'au moins un agent tensioactif lipophile, d'au moins un agent tensioactif hydrophile et d'au moins un lipide amphiphile ionique conférant à l'émulsion un pH allant de 5,5 à 7,5, les globules huileux enrobés ayant un diamètre moyen inférieur à 500 nanomètres.

Au sens de la présente invention, on entend par composé actif lipophile le composé actif en soi lorsqu'il est lui-même une huile ou bien, s'il ne l'est pas, le composé actif dissous dans une huile. Les huiles pouvant être utilisées sont les huiles servant classiquement de support dans les compositions cosmétiques comme par exemple les triglycérides d'acides gras à chaînes courtes et les huiles de silicone.

L'invention permet de disposer d'émulsions particulièrement stables, ayant des gouttelettes de phase grasse de taille extrèmement petite, revêtues d'une couche mono ou oligo lamellaire extrèmement fine. On entend par couche oligolamellaire une couche comprenant de 2 à 5 lamelles lipidiques. La taille moyenne des globules huileux revêtus est inférieure à 500 nanomètres, et de préférence à 200 nanomètres, pour les formulations de type lait ou crème, et elle est inférieure à 80 nanomètres pour les formulations transparentes ou opalescentes. Du fait de la petite taille des globules huileux, leur pénétration dans les espaces intercornéocytaires qui sont de taille comparable est grandement facilitée.

L'actif contenu ou constitué par le globule huileux peut de ce fait être véhiculé et délivré dans la peau ou le cheveu là où son action sera la plus efficace.

Selon un mode de réalisation préférentiel de l'invention, l'agent tensioactif lipophile et l'agent tensioactif hydrophile comportent chacun au moins une chaîne grasse saturée ayant plus de 12 atomes de carbone environ. Plus préférentiellement encore, cette chaîne grasse contient de 16 à 22 atomes de carbone.

Selon un autre mode de réalisation préférentiel de l'invention, l'agent tensioactif lipophile présente un HLB compris entre environ 2 et environ 5. Comme cela est bien connu, on entend par HLB (Hydrophilic-Lipophilic Balance) l'équilibre entre la dimension et la force du groupe hydrophile et la dimension et la force de groupe lipophile de l'agent tensioactif.

Des exemples de tels agents tensioactifs lipophiles sont le distéarate de sucrose, le distéarate de diglycéryle, le tristéarate de tétraglycéryle, le décastéarate de décaglycéryle, le monostéarate de diglycéryle, le tristéarate d'hexaglycéryle, le pentastéarate de décaglycéryle, le monostéarate de sorbitane, le tristéarate de sorbitane, le monostéarate de diéthylène glycol, l'ester de glycérol et d'acides palmitique et stéarique, le monostéarate polyoxyéthylèné 2 OE (comportant 2 motifs oxyéthylène), le mono et dibéhénate de glycéryle, le tétrastéarate de pentaérythritol.

L'agent tensioactif hydrophile présente de préférence un HLB compris entre environ 8 et environ 12.

On peut citer comme exemples de tels tensioactifs hydrophiles les composés suivants : le monostéarate de sorbitane polyoxyéthyléné 4 OE, le tristéarate de sorbitane polyoxyéthyléné 20 OE, le monostéarate polyoxyéthyléné 8 OE, le monostéarate d'hexaglycéryle, le monostéarate polyoxyéthyléné 10 OE, le distéarate polyoxyéthylèné 12 OE et le distéarate de méthylglucose polyoxyéthyléné 20 OE.

Le lipide amphiphile ionique mis en oeuvre dans le cadre de la présente invention est de préférence choisi parmi le groupe comprenant les lipides anioniques neutralisés, les lipides amphotères et les dérivés alkylsulfoniques.

Les lipides anioniques neutralisés sont choisis en particulier parmi :

- les sels alcalins du dicétylphosphate, et en particulier les sels de sodium et de potassium ;
- les sels alcalins du dimyristylphosphate, et en particulier les sels de sodium et de potassium ;
- les sels alcalins du cholestérol sulfate, et en particulier le sel de sodium ;
- les sels alcalins du cholestérol phosphate, et en particulier le sel de sodium ;
- les sels monosodique et disodique des acides acylglutamiques, et en particulier les sels monosodique et disodique de l'acide N-stéaroyl glutamique,
- le sel de sodium de l'acide phosphatidique.

Les lipides amphotères sont choisis en particulier parmi les phospholipides et notamment la phosphatidyléthanolamine de soja pure.

Les dérivés alkylsulfoniques sont avantageusement les composés de formule :

$$\begin{array}{l} R\text{-}CH\text{-}CO\text{-}O\text{-}(CH_2CH_2O)_2\text{-}CH_3 \\ \quad\ | \\ \quad SO_3M \end{array}$$

dans laquelle R représente les radicaux $C_{16}H_{33}$ et $C_{18}H_{37}$ pris en mélange ou séparément et M est un métal alcalin, de préférence le sodium.

L'enrobage selon l'invention des globules huileux demande de préférence l'utilisation d'une quantité totale d'agent tensioactif hydrophile, d'agent tensioactif lipophile et de lipide amphiphile ionique comprise entre environ 2% et environ 6% en poids par rapport au poids total de la composition. Encore plus préférentiellement, cette quantité est comprise entre 3% et 4%. Les quantités relatives de tensioactifs lipophile, hydrophile et de lipide amphiphile ionique varient de préférence dans les fourchettes respectives suivantes : 35-55% / 25-40% / 15-35% en poids par rapport à leur poids total.

La phase grasse, c'est à dire les gouttelettes huileuses enrobées, représente de préférence 5 à 50 % en poids par rapport au poids total de la composition. Encore plus préférentiellement, ce pourcentage est compris entre 10 et 40 %. De préférence, le rapport huile/eau est égal ou inférieur à 1.

Le rapport pondéral des globules huileux aux éléments constitutifs de l'enrobage est de préférence de 2 à 13 ; encore plus préférentiellement ce rapport est égal à 7 environ.

Lorsque les compositions selon l'invention sont utilisées pour le traitement cosmétique de la peau ou dans un but dermatologique, l'actif contenu dans la phase huileuse est, par exemple, choisi parmi les agents antioxydants, anti-radicaux libres, hydratants, mélanorégulateurs, accélérateurs de bronzage, dépigmentants, de coloration de la peau, liporégulateurs, amincissants, antiacnéiques, antiséborrhéiques, anti-vieillissement, anti-rides, anti-UV, kératolytiques, anti-inflammatoires, rafraichissants, cicatrisants, protecteurs vasculaires, antibactériens, antifongiques, antiperspirants, déodorants, conditionneurs de la peau, immunomodulateurs, nourrissants et les huiles essentielles et les parfums.

Lorsque les compositions selon l'invention sont utilisées pour le traitement cosmétique de la matière kératinique, l'actif contenu dans la phase huileuse est, par exemple, choisi parmi les agents mélanorégulateurs, liporégulateurs, antiséborrhéiques, anti-vieillissement, anti-UV, kératolytiques, antibactériens, antifongiques, antipelliculaires, anti-chute des cheveux, colorants capillaires, décolorants capillaires, réducteurs pour permanente, conditionneurs des cheveux et nourrissants.

On peut citer comme exemples d'actifs lipophiles pour le traitement de la peau et/ou des cheveux, utilisables dans le cadre de la présente invention les composés suivants :

D $\alpha$ tocophérol, DL $\alpha$ tocophérol, acétate de D $\alpha$ tocophérol, acétate DL $\alpha$ tocophérol, palmitate d'ascorbyle, glycérides de vitamine F, vitamines D et notamment vitamine $D_2$ et vitamine $D_3$, rétinol, esters de rétinol (palmitate de rétinol, propionate de rétinol), $\beta$ carotène, D panthénol, farnésol, acétate de farnésyle, huiles riches en acides gras essentiels et notamment huiles de jojoba et de cassis, acide n octanoyl-5 salicylique, acide salicylique, alkylesters d'$\alpha$-hydroxyacides tels que acide citrique, acide lactique et acide glycolique, acide asiatique, acide madécassique, asiaticoside, extrait total de Centella Asiatica, acide $\beta$ glycyrrhétinique, $\alpha$ bisabolol, céramides et notamment le 2 oléoylamino-1,3 octadécane, phytanetriol, sphingomyéline de lait, phospholipides d'origine marine riches en acides gras essentiels polyinsaturés, éthoxyquine, extrait de romarin, extrait de mélisse, quercetine, extrait de microalgues séchées (algoxan red de Algatec), huile essentielle de bergamotte, octylmethoxycinnamate (Parsol MCX - Givaudan-Roure), butylméthoxydibenzoylméthane (Parsol 1789 - Givaudan-Roure), octyl triazone (Uvinul T150 - BASF), oxydes de fer jaune, brun, noir, rouge, oxydes de titane, pouvant se présenter sous forme micrométrique ou nanométrique ou sous forme enrobée (perfluoroalkyl), di-tertiobutyl-3,5 hydroxy-4 benzylidène-3 camphre, 2-benzotriazol -2-yl- 4-méthyl-6-[3-[1,3,3,3-tétraméthyl-1 -[(triméthylsilyl)oxy]-disiloxanyl]-2-méthyl-propyl]phénol, huile perfluorée (perfluorodécaline, perfluorooctyl bromure), huile de maïs hyperoxygénée (Epaline 100 commercialisée par la société Carilene).

Par ailleurs, la demanderesse a trouvé que l'incorporation de pigments de taille nanométrique, et notamment de nanotitanes, dans les compositions de l'invention permettait d'obtenir des compositions de protection solaire ayant un indice de protection (SPF) plus élevé que celui des compositions ne comportant pas les globules selon l'invention. Par taille nanométrique, il faut entendre une taille de particules inférieure à 200 nanomètres, et notamment de l'ordre de 10 à 50 nanomètres.

Les compositions selon la présente invention peuvent en outre contenir dans la phase aqueuse un ou plusieurs composés hydrophiles cosmétiquement ou dermatologiquement actifs, libres ou encapsulés.

On peut utiliser les actifs hydrophiles classiquement utilisés comme agents antioxydants, anti-radicaux libres, hydratants, mélanorégulateurs, accélérateurs de bronzage, dépigmentants, de coloration de la peau, liporégulateurs, amincissants, anti-acnéiques, antiséborrhéiques, anti-vieillissement, anti-rides, anti-UV (acide benzène-1,4-[di(3-méthylidènecampho-10-sulfonique)], kératolytiques, anti-inflammatoires, rafraichissants, cicatrisants, protecteurs vasculaires, antibactériens, antifongiques, antiperspirants, déodorants, conditionneurs de la peau et du cheveu, immunomodulateurs, nourrissants, antipelliculaires, anti-chute des cheveux, colorants capillaires, décolorants capillaires, réducteurs pour permanente et les huiles essentielles et les parfums.

Comme actif hydrophile, on peut en particulier utiliser un alcool à chaîne courte (en $C_1$ à $C_6$) tel que l'éthanol, un polyol tel qu'un glycol, et notamment le 1,3-butylène glycol, le propylène glycol, le dipropylène glycol, l'isoprène glycol ou l'hexylène glycol, la glycérine, la polyglycérine et le sorbitol.

Lorsqu'il se présente à l'état encapsulé, ce composé peut être incorporé dans un vésicule lipidique obtenu à partir de lipides ioniques, non ioniques ou d'un mélange des deux. Il peut encore être incorporé dans des nanoparticules lipidiques telles que les nanosphères, nanoéponges ou nanocapsules.

L'incorporation de vésicules lipidiques dans les compositions de l'invention est particulièrement avantageuse du fait de la complémentarité et de la bonne compatibilité de ces deux types de véhicules que sont, d'une part, les globules huileux contenant des actifs lipophiles, de taille moyenne préférée voisine de 200 nm, délimités par leur enrobage cristal liquide lamellaire et, d'autre part, les vésicules lipidiques à coeur aqueux contenant des actifs hydrophiles, de taille moyenne préférée voisine de 200 nm, délimités par leur paroi lamellaire.

Les inventeurs ont de plus constaté que les vésicules lipidiques à base de lipides ioniques naturels insaturés, qui sont particulièrement sensibles à la présence de tensioactifs en phase aqueuse (ce qui est le cas dans les émulsions classiques) et à celle des peroxydes étaient particulièrement bien conservées dans les compositions de l'invention à base de tensioactifs à chaînes grasses saturées ayant plus de 12 atomes de carbone.

Les compositions de l'invention peuvent encore contenir dans la phase aqueuse divers additifs complémentaires tels que des agents conservateurs, des agents séquestrants, des agents gélifiants.

Selon la viscosité que l'on souhaite obtenir pour la composition finale, on ajoute ou non un ou plusieurs gélifiants. Ainsi, lorsque l'on veut obtenir des compositions sous forme de sérums, on n'introduit pas de gélifiant dans la composition.

Les compositions de l'invention peuvent encore contenir dans la phase grasse divers additifs complémentaires tels que huiles, cires ou gommes ayant par exemple des propriétés émollientes ou lubrifiantes.

Les compositions se présentent le plus fréquemment sous forme de lait, crème ou gel, d'autres modes de présentation n'étant pas exclus. Elles sont transparentes ou translucides quand la taille des particules est inférieure à 80 nm.

Un autre objet de l'invention est un procédé de préparation des compositions ci-dessus décrites caractérisé en ce que, dans une première étape, on mélange sous agitation la phase grasse comprenant le tensioactif lipophile, le tensioactif hydrophile, le lipide amphiphile ionique, le composé actif au plan cosmétique ou dermatologique et la phase aqueuse et, dans une deuxième étape, on soumet le mélange obtenu à une homogénéisation basée sur le principe de la cavitation.

Dans la première étape, le mélange est soumis à agitation classique par exemple dans un homogénéisateur tournant à une vitesse comprise entre 500 et 5000 t/mn environ pendant un temps compris entre 10 et 60 mn environ à une température comprise entre 20 et 95°C environ.

L'homogénéisation basée sur le principe de la cavitation de la deuxième étape est une étape clé du procédé selon l'invention. Cette homogénéisation résulte du phénomène de cavitation créé et entretenu au sein du mélange, alors sous forme liquide, en mouvement à une vitesse linéaire d'au moins 100m/s.

Elle peut être opérée par utilisation d'un homogénéisateur haute pression fonctionnant sous des pressions comprises entre 200 et 1500 bars environ. Le principe d'utilisation de ce type d'homogénéisateur est bien connu de l'homme de l'art. On opère par passages successifs, généralement de 2 à 10 passages, sous la pression retenue, le mélange étant ramené à pression normale entre chaque passage.

L'homogénéisation de la deuxième étape peut également être obtenue sous l'action d'ultrasons ou encore par utilisation d'homogénéisateurs équipés d'une tête de type rotor-stator.

Lorsque la phase aqueuse contient des actifs hydrophiles cosmétiques ou dermatologiques, si ces derniers sont introduits à l'état libre, ils sont introduits dans la première étape. Si par contre ils sont introduits à l'état encapsulé, ils doivent être introduits dans une troisième étape ultérieure. Ils le sont alors par simple mélange.

L'invention va être maintenant plus complètement décrite, dans ses objets et ses caractéristiques, à l'aide des exemples qui vont suivre.

On opère dans les exemples en mettant en oeuvre le mode opératoire suivant :

La phase huileuse A1 et la phase aqueuse B sont chauffées séparément à la température de 80° C.

Sous l'agitation de 4000t/mn fournie par un homogénéisateur Moritz de type Turbo Lab 2100, on verse la phase B sur la phase A1 et l'on conserve ces conditions d'agitation et de température pendant 30 minutes.

Le mélange est alors introduit dans un homogénéisateur haute pression Soavi de type OBL réglé à la pression de 500 bars pour les exemples 1 à 7, et à la pression de 1200 bars pour l'exemple 8, pour 3 passages consécutifs.

On obtient ainsi une émulsion huile dans eau stabilisée, dont les globules huileux ont une taille moyenne inférieure à 200 nm et une polydispersité d'indice inférieure à 0,1 telles que mesurées par un granulomètre à laser de type AMTECH BI 90.

On refroidit ensuite l'émulsion pour la ramener à température ambiante, ce qui prend environ 60 minutes. On ajoute alors à l'émulsion la phase huileuse A2 et on soumet le tout à l'agitation donnée par le Turbo Lab 2100 à la vitesse de 3000 t/mn pendant 10 mn, après quoi on introduit ce prémélange dans le Soavi - OBL réglé à la pression de 350 bars pour 2 passages supplémentaires.

Après chacun de ces deux passages, on refroidit le produit jusqu'à retour à la température ambiante.

A cette émulsion A1 + B + A2 , on ajoute la phase C lorqu'elle est présente, et l'on agite le tout à l'aide d'un homogénéisateur Rayneri équipé d'une turbine de type défloculeuse à la vitesse de 2500 t/mn pendant 30 mn à la température ambiante.

**EXEMPLE 1 : LAIT HYDRATANT POUR LE CORPS**

*Phase A1 :*

| | |
|---|---|
| - Distéarate de sucrose commercialisé par la Société Stéarinerie Dubois | 1,5 % |
| - Stéarate de sorbitane oxyéthyléné à 4 moles d'oxyde d'éthylène commercialisé par la Société ICI sous le nom de "TWEEN 61" | 1 % |
| - Sel disodique de l'acide N-stéaroyl glutamique commercialisé par la société Ajinomoto sous le nom de ⟨⟨ACYLGLUTAMATE HS 21 ⟩⟩ | 0,75 % |
| - Stearyl heptanoate | 3 % |
| - Vaseline codex | 1 % |
| - Huile de silicone volatile | 2 % |
| - Huile de jojoba | 2 % |
| - Acétate de vitamine E | 0,5 % |

*Phase A2 :*

| | |
|---|---|
| - Gomme de silicone commercialisée par la Société Dow Corning sous le nom ⟨⟨$Q_2$-1403 FLUID ⟩⟩ | 2 % |
| - Propylparaben | 0,1 % |
| - Parfum | 0,3 % |

*Phase B :*

| | |
|---|---|
| - Glycérine | 5 % |
| - Méthylparaben | 0,3 % |
| - Propylène glycol | 3 % |
| - Eau déminéralisée | qsp 100 % |

*Phase C :*

| | |
|---|---|
| - Mélange de polymères carboxyvinyliques commercialisé sous le nom ⟨⟨CARBOPOL 940 ⟩⟩par la Société Goodrich | 0,3 % |
| - Triéthanolamine | 0,1 % |
| - Eau déminéralisée | 9,6 % |

La taille moyenne des globules d'huile de l'émulsion stabilisée est de 160 nm avec un indice de polydispersité de 0,07.

**EXEMPLE 2 : CREME DE JOUR ANTI-AGE POUR LE VISAGE**

*Phase A1 :*

| | |
|---|---|
| - Distéarate de diglycéryle commercialisé par la Société Nihon Emulsion sous la référence ⟨⟨ EMALEX PSGA ⟩⟩ | 1,75 % |
| - Distéarate de méthylglucose polyoxyéthyléné 20 OE commercialisé par la Société Amerchol sous le nom ⟨⟨GLUCAM E 20 DISTEARATE ⟩⟩ | 1,15 % |
| - Dicétylphosphate de sodium | 0,75 % |
| - Stearyl heptanoate | 4 % |
| - Vaseline codex | 1,5 % |
| - Huile d'avocat | 3,2 % |
| - Huile de jojoba | 3 % |
| - Huile de silicone volatile | 2,7 % |
| - Acétate de vitamine E | 1 % |
| - D $\alpha$ tocophérol naturel commercialisé par la Société Henkel sous le nom ⟨⟨COPHEROL 1300 ⟩⟩ | 1 % |
| - Glycérides de Vitamine F | 3 % |
| - Palmitate de rétinol commercialisé par la société Fluka, dosé à 1500 UI/mg | 0,5 % |

*Phase A2 :*

| | |
|---|---|
| - Gomme de silicone commercialisée par Dow Corning sous le nom ⟨⟨Q2-1403 FLUID ⟩⟩ | 3 % |
| - Propylparaben | 0,2% |
| - Parfum | 0,3% |

*Phase B :*

| | |
|---|---|
| - Glycérine | 3 % |
| - Hydroxyproline | 1 % |
| - D-panthenol | 1 % |
| - Méthylparaben | 0,3 % |
| - Eau déminéralisée | qsp 100 % |

*Phase C :*

| | |
|---|---|
| - Mélange de polymères carboxyvinyliques commercialisé sous le nom ⟨⟨CARBOPOL 940 ⟩⟩par la Société Goodrich | 0,4 % |
| - Eau déminéralisée | 9,5 % |
| - Triéthanolamine | 0,25 % |

La taille moyenne des globules d'huile de l'émulsion stabilisée est de 190 nm avec un indice de polydispersité de 0,07.

On obtient une crème lisse, blanche, souple, fraîche et très confortable à l'application.

**EXEMPLE 3 : CREME DE NUIT AUX LIPOSOMES NON IONIQUES POUR PEAUX STRESSEES ET DESORGANISEES**

*Phase A1* :

| | |
|---|---|
| - Tristéarate de tétraglycéryle commercialisé par la Société Nikkol sous le nom ⟨⟨TETRAGLYN 3 S⟩⟩ | 2 % |
| - Stéarate de sorbitane oxyéthylėné à 4 moles d'oxyde d'éthylène commercialisé par la Société ICI sous le nom de ⟨⟨TWEEN 61⟩⟩ | 1,4 % |
| - Dicétylphosphate de sodium | 1 % |
| - Stearyl heptanoate | 5,5 % |
| - Vaseline codex | 2,1 % |
| - Huile de macadamia | 4,5 % |
| - Huile d'amande d'abricot | 3,5 % |
| - Huile de silicone volatile | 3,7 % |
| - Acétate de Vitamine E | 1 % |
| - Glycérides de Vitamine F | 3 % |
| - D $\alpha$ tocophérol naturel commercialisé par Henkel sous la référence ⟨⟨COPHEROL 1300⟩⟩ | 0,5 % |

*Phase A2* :

| | |
|---|---|
| - Gomme de silicone commercialisée par Dow Corning sous le nom ⟨⟨$Q_2$-1403 FLUID⟩⟩ | 4 % |
| - Propylparaben | 0,1% |
| - Parfum | 0,3% |

*Phase B* :

| | |
|---|---|
| - Méthylparaben | 0,3 % |
| - Eau déminéralisée | qsp 100 % |

<u>Phase C</u> : Elle se décompose ici en deux phases C1 et C2

*<u>Phase C1</u> : Phase vésiculaire*

| | |
|---|---|
| - Tristéarate de tétraglycéryle commercialisé par Nikkol sous le nom ⟨⟨TETRAGLYN 3 S⟩⟩ | 0,46 % |
| - Cholestérol | 0,46 % |
| - Sel monosodique de l'acide N-stéaroyl glutamique commercialisé sous le nom ⟨⟨ ACYLGLUTAMATE HS 11⟩⟩par la Société Ajinomoto | 0,08 % |
| - Glycérine | 3 % |
| - Hydroxyproline | 1 % |
| - Eau déminéralisée | 5 % |

Cette phase est préparée de la façon suivante :
Les 3 lipides constituant la paroi lipidique des vésicules sont chauffés à la température de 115° nécessaire et suffisante pour en réaliser la co-fusion. On obtient ainsi un mélange liquide transparent que l'on refroidit à la température de 90° C.

On ajoute alors le reste de la phase aqueuse C1 pour réaliser l'hydratation du mélange lipidique à cette même température de 90° C par agitation lente pendant 60 minutes. On refroidit ce mélange à 60° C que l'on introduit alors dans l'homogénéisateur Soavi de type OBL réglé à la pression de 500 bars pour 3 passages successifs.

*<u>Phase C2</u> :*

| | |
|---|---|
| - Mélange de polymères carboxyvinyliques commercialisé sous le nom de ⟨⟨CARBOPOL 940⟩⟩par la Société GOODRICH | 0,3 % |
| - Triéthanolamine | 0,1 % |
| - Eau déminéralisée | 9,6 % |

La phase C1, refroidie à la température ambiante, est mélangée à la phase gélifiée C2 pour constituer la phase C.

La taille moyenne des globules d'huile de l'émulsion stabilisée est de 180 nm et l'indice de polydispersité est de 0,07.

On obtient une crème blanche, brillante, lisse et de grand confort après application.

**EXEMPLE 4 : CREME DE JOUR AUX LIPOSOMES POUR PEAUX SENSIBLES, STRESSEES, DESORGANISEES.**

*Phase A1* :

| | |
|---|---|
| - Monostéarate de diglycéryle commercialisé par la Société Nikkol sous la référence 〈〈DGMS 〉〉 | 1,5 % |
| - Monostéarate polyoxyéthyléné 8 OE commercialisé par ICI sous le nom de 〈〈Myrj 45 〉〉 | 1 % |
| - Sel monosodique de l'acide N-stéaroyl glutamique commercialisé par la société Ajinomoto sous le nom 〈〈ACYLGLUTAMATE HS 11 〉〉 | 0,75 % |
| - Stearyl heptanoate | 4 % |
| - Vaseline codex | 1 % |
| - Silicone volatile | 3,2 % |
| - Huile de jojoba | 3 % |
| - Huile d'amande douce | 2,7 % |
| - Acétate de vitamine E | 0,5 % |
| - D $\alpha$ tocophérol naturel commercialisé par la Société Henkel sous le nom 〈〈COPHEROL 1300 〉〉 | 1 % |
| - Octylméthoxycinnamate commercialisé par la Société Givaudan sous le nom 〈〈PARSOL MCX 〉〉 | 2 % |
| - Butylméthoxydibenzoylméthane commercialisé par la Société Givaudan sous le nom 〈〈PARSOL 1789 〉〉 | 0,5 % |

*Phase A2* :

| | |
|---|---|
| - Gomme de silicone commercialisée par la Société Dow Corning sous le nom 〈〈$Q_2$-1403 FLUID 〉〉 | 3 % |
| - Conservateur | 0,1% |
| - Parfum | 0,3% |

*Phase B* :

| | |
|---|---|
| - Conservateurs | 0,1 % |
| - Parfum | 0,3 % |
| - Triéthanolamine | 0,35 % |
| - Eau déminéralisée | qsp 100 % |

Phase C : Elle se compose de deux phases C1 et C 2 :

*Phase C1 : Phase vésiculaire*

| | |
|---|---|
| - Mélange de phospholipides dans un mélange eau/alcool, commercialisé sous le nom 〈〈NATIPIDE II 〉〉par la Société Natterman Phospholipid | 5 % |
| - Glycérine | 3 % |
| - Eau déminéralisée | 9 % |

Cette phase est préparée en dispersant le NATIPIDE II à température ambiante à l'aide d'un barreau aimanté tournant à la vitesse de 300 t/mn pendant 30 mn dans le reste de la phase aqueuse.

*Phase C2* :

| | |
|---|---|
| - Polymère carboxyvinylique commercialisé par la Société SIGMA sous le nom "SYNTHALEN K" | 0,5 % |
| - Eau déminéralisée | 9,3 % |
| - Triéthanolamine | 0,2 % |

La phase C1 est alors mélangée à la phase C2 pour constituer la phase C.

La taille moyenne des globules d'huile de l'émulsion stabilisée est de 160 nm et l'indice de polydispersité est de 0,08.

On obtient une crème blanche, lisse à texture fine, non grasse et très confortable.

**EXEMPLE 5 : LAIT PARFUME POUR LE CORPS**

*Phase A* :

| | |
|---|---|
| - Monostéarate de diglycéryle commercialisé par la Société Nikkol sous le nom ⟨⟨DGMS⟩⟩ | 1,5 % |
| - Stéarate de sorbitane oxyéthyléné à 4 moles d'oxyde d'éthylène commercialisé par la Société ICI sous le nom ⟨⟨TWEEN 61⟩⟩ | 1 % |
| - Sel disodique de l'acide N-stéaroyl glutamique commercialisé par la société Ajinomoto sous le nom de ⟨⟨ACYLGLUTAMATE HS 21⟩⟩ | 0,75 % |
| - Stéaryl heptanoate | 2 % |
| - Huile de sésame | 6 % |
| - Huile de silicone volatile | 2 % |
| - Huile essentielle de bergamotte (exempte de Bergaptène) | 8 % |

*Phase B* :

| | |
|---|---|
| - Gomme de silicone commercialisée par la Société Dow Corning sous le nom ⟨⟨$Q_2$-1403 FLUID⟩⟩ | 2 % |
| - Conservateur | 0,1 % |

*Phase C* :

| | |
|---|---|
| - Glycérine | 3 % |
| - Propylène glycol | 5 % |
| - Conservateurs | 0,3 % |
| - Eau déminéralisée | qsp 100 % |

La taille moyenne des globules d'huile de l'émulsion stabilisée est de 130 nm avec un indice de polydispersité de 0,06.

On obtient un lait blanc, très fluide, pulvérisable à l'aide d'un flacon-pompe. Les propriétés parfumantes de ce lait sont rémanentes dans le temps.

**EXEMPLE 6 : CREME TEINTEE DESTINEE AUX PEAUX CLAIRES**

Phase A1:

| | |
|---|---|
| - Distéarate de sucrose commercialisé par la Société Stearinerie Dubois | 2 % |
| - Stéarate de sorbitane oxyéthyléné à 4 moles d'oxyde d'éthylène commercialisé par la Société ICI sous le nom 〈〈TWEEN 61 〉〉 | 1,35 % |
| - Dicétylphosphate de sodium | 1 % |
| - Stearyl heptanoate | 5,5 % |
| - Vaseline codex | 2,1 % |
| - Huile de silicone volatile | 6 % |
| - Huile d'avocat | 4 % |
| - Huile de jojoba | 4 % |
| - Acétate de DL $\alpha$ tocophérol | 0,5 % |

Phase A2 :

| | |
|---|---|
| - Gomme de silicone commercialisée par la Société Dow Corning sous le nom 〈〈$Q_2$-1403 FLUID 〉〉 | 4 % |
| - Propylparaben | 0,1% |
| - Parfum | 0,3% |

Phase B :

| | |
|---|---|
| - Conservateurs | 0,4 % |
| - Sel pentasodique de l'acide amino(triméthylène phosphonique) commercialisé par la société Monsanto sous le nom 〈〈Dequest 2046 〉〉(séquestrant) | 0,05% |
| - Glycérine | 3 % |
| - Eau déminéralisée | 45 % |

Phase C :

| | |
|---|---|
| - Saponite commercialisée par la Société Vanderbilt sous le nom "VEEGUM" | 0,35 % |
| - Oxydes de fer jaunes | 0,77 % |
| - Oxydes de fer bruns | 0,77 % |
| - Oxydes de fer noirs | 0,35 % |
| - Dioxydes de titane | 5,11 % |
| - Gomme de xanthane commercialisée par la Société Kelco sous le nom "KELTROL T" | 0,2 % |
| - Eau déminéralisée | qsp 100 % |

La taille moyenne des globules d'huile enrobés est de 180 nm avec un indice de polydispersité de 0,08.
On obtient ainsi une crème teintée, lisse, couvrant bien uniformément les imperfections du teint du visage.

**EXEMPLE 7 : CREME DE PROTECTION SOLAIRE A FORT INDICE DE PROTECTION**

*Phase A1* :

| | |
|---|---|
| - Distéarate de sucrose commercialisé par la Société Stearinerie Dubois | 1,5 % |
| - Stéarate de sorbitane oxyéthyléné à 4 moles d'oxyde d'éthylène commercialisé par la Société ICI sous le nom "TWEEN 61" | 1 % |
| - Sel disodique de l'acide N-stéaroyl glutamique commercialisé par la société Ajinomoto sous le nom de 《 ACYLGLUTAMATE HS 21 》 | 0,85 % |
| - Stearyl heptanoate | 5,5 % |
| - Vaseline codex | 2,1 % |
| - Huile d'avocat | 4 % |
| - Huile de jojoba | 4 % |
| - Acétate de DL $\alpha$ tocophérol | 0,5 % |
| - Octyl méthoxycinnamate commercialisé par la Société Givaudan Roure sous le nom "PARSOL MCX" | 2 % |
| - Butylméthoxycinnamate commercialisé par la Société Givaudan Roure sous le nom de "PARSOL 1789" | 0,5 % |
| - Ethoxyquine | 0,03 % |

*Phase A2* :

| | |
|---|---|
| - Dioxyde de titane nanométrique commercialisé par la Société Tayca sous le nom "MT 100 T" | 10 % |
| - Dodécaméthyl cyclohexasiloxane commercialisé par la Société Hercules | 10 % |
| - Gomme de silicone commercialisée par la Société Dow Corning sous le nom "$Q_2$-1403 Fluid" | 4 % |

*Phase B* :

| | |
|---|---|
| - Conservateurs | 0,4 % |
| - Sequestrant | 0,05 % |
| - Glycérine | 3,0 % |
| - Eau déminéralisée | 42,67 % |

*Phase C* :

| | |
|---|---|
| - Mélange de polymères carboxyvinyliques commercialisé par la Société Goodrich sous le nom "CARBOPOL 940" | 0,3 % |
| - Triéthanolamine | 0,1 % |
| - Eau déminéralisée | qsp 100 % |

La taille moyenne des globules d'huile enrobés est de 170 nm avec un indice de polydispersité de 0,12.

On obtient une crème blanche, lisse, qui a un meilleur indice de protection solaire que la même composition dans lesquels les globules huileux ne sont pas enrobés selon l'invention.

**EXEMPLE 8 : COMPOSITION TRANSPARENTE POUR L'HYDRATATION DE LA PEAU**

*Phase A* :

| | |
|---|---|
| - Distéarate de diglycéryle commercialisé par la Société Nihon Emulsion sous la référence ⟨⟨EMALEX PSGA⟩⟩ | 2,15 % |
| - Distéarate de méthylglucose polyoxyéthyléné 20 OE commercialisé par la Société Amerchol sous le nom ⟨⟨GLUCAM E 20 DISTEARATE⟩⟩ | 1,15 % |
| - Sel disodique de l'acide N-stéaroyl glutamique commercialisé par la société Ajinomoto sous le nom de ⟨⟨ACYLGLUTAMATE HS 21⟩⟩ | 0,85 % |
| - Stéaryl heptanoate | 2 % |
| - Huile d'avocat | 6 % |
| - Huile de jojoba | 6 % |
| - Huile de silicone volatile | 4 % |
| - Acétate de vitamine E | 1 % |
| - Acide stéarique | 0,6 % |

*Phase B* :

| | |
|---|---|
| - Glycérine | 5 % |
| - Méthylparaben | 0,3 %- |
| - Ethanol | 15 % |
| - Eau déminéralisée | qsp 100 % |

On obtient une composition transparente dont la taille moyenne des globules d'huile de l'émulsion stabilisée est de 50 nm avec un indice de polydispersité de 0,09.

**Revendications**

1. Composition cosmétique ou dermatologique constituée par une émulsion du type huile dans eau formée de globules huileux pourvus chacun d'un enrobage cristal liquide lamellaire et dispersés dans une phase aqueuse, caractérisée en ce que chaque globule huileux contenant au moins un composé lipophile actif cosmétique ou dermatologique est unitairement enrobé d'une couche monolamellaire ou oligolamellaire obtenue à partir d'au moins un agent tensioactif lipophile, d'au moins un agent tensioactif hydrophile et d'au moins un lipide amphiphile ionique conférant à l'émulsion un pH allant de 5,5 à 7,5, les globules huileux enrobés ayant un diamètre moyen inférieur à 500 nanomètres.

2. Composition selon la revendication 1, caractérisée en ce que les globules huileux ont un diamètre moyen inférieur à 200 nanomètres.

3. Composition selon l'une quelconque des revendications précédentes, caractérisée en ce que l'agent tensioactif lipophile et l'agent tensioactif hydrophile comportent chacun au moins une chaine grasse saturée ayant plus de 12 atomes de carbone environ, de préférence entre 16 et 22.

4. Composition selon l'une quelconque des revendications précédentes, caractérisée en ce que l'agent tensioactif lipophile présente un HLB (balance hydrophile-lipophile) compris entre 2 et 5.

5. Composition selon la revendication 4, caractérisée en ce que l'agent tensioactif lipophile présentant un HLB compris entre 2 et 5 est choisi parmi le distéarate de sucrose, le distéarate de diglycéryle, le tristéarate de tétraglycéryle, le décastéarate de décaglycéryle, le monostéarate de diglycérol, le tristéarate d'hexaglycéryle, le

pentastéarate de décaglycéryle, le monostéarate de sorbitane, le tristéarate de sorbitane, le monostéarate de diéthylène glycol, l'ester de glycérol et d'acides palmitique et stéarique, le monostéarate polyoxyéthyléné 2 OE, (comprenant 2 motifs d'oxyde d'éthylène), le mono et dibéhénate de glycéryle, le tétrastéarate de pentaérythritol.

6. Composition selon l'une quelconque des revendications précédentes, caractérisée en ce que l'agent tensioactif hydrophile présente un HLB compris entre 8 et 12.

7. Composition selon la revendication 6, caractérisée en ce que l'agent tensioactif hydrophile présentant un HLB compris entre 8 et 12 est choisi parmi le monostéarate de sorbitane polyoxyéthyléné 4 OE, le tristéarate de sorbitane polyoxyéthyléné 20 OE, le monostéarate polyoxyéthyléné 8 OE, le monostéarate d'hexaglycéryle, le monostéarate polyoxyéthyléné 10 OE, le distéarate polyoxyéthyléné 12 OE, le distéarate de méthylglucose polyoxyéthyléné 20 OE.

8. Composition selon l'une quelconque des revendications précédentes, caractérisée en ce que le lipide amphiphile ionique est choisi parmi le groupe comprenant les lipides anioniques neutralisés, les lipides amphotères et les dérivés alkylsulfoniques.

9. Composition selon la revendication 8, caractérisée en ce que le lipide amphiphile ionique est choisi parmi les sels alcalins du dicétylphosphate, les sels alcalins du dimyristylphosphate, les sels alcalins du cholestérol sulfate, les sels alcalins du cholestérol phosphate, les sels mono et disodiques des acides acylglutamiques, les phospholipides, les dérivés alkylsulfoniques de formule :

$$\begin{array}{l} R\text{-}\underset{\displaystyle SO_3M}{\underset{|}{CH}}\text{-}CO\text{-}O\text{-}(CH_2CH_2O)_2\text{-}CH_3 \end{array}$$

dans laquelle R représente les radicaux $C_{16}H_{33}$ et $C_{18}H_{37}$ pris en mélange ou séparément et M est un métal alcalin.

10. Composition selon l'une quelconque des revendications précédentes, caractérisée en ce que l'agent tensioactif lipophile, l'agent tensioactif hydrophile et le lipide amphiphile ionique sont présents en une quantité comprise entre 2 et 6 % en poids par rapport au poids total de la composition et de préférence entre 3 et 4 %.

11. Composition selon l'une quelconque des revendications précédentes, caractérisée en ce que l'agent tensioactif lipophile, l'agent tensioactif hydrophile et le lipide amphiphile ionique varient de préférence dans les fourchettes respectives de 35-55%, 25-40% et 15-35% en poids par rapport à leur poids total.

12. Composition selon l'une quelconque des revendications précédentes, caractérisée en ce que les globules huileux enrobés représentent de 5 à 50% en poids par rapport au poids total de la composition, de préférence de 10 à 40%.

13. Composition selon l'une quelconque des revendications précédentes, caractérisée en ce que le rapport pondéral des globules huileux aux tensioactifs constitutifs de l'enrobage est compris entre 2 et 13, de préférence égal à 7 environ.

14. Composition selon l'une quelconque des revendications précédentes, caractérisée en ce que la phase aqueuse contient en outre un ou plusieurs composés hydrophiles cosmétiquement ou dermatologiquement actifs, libres ou encapsulés.

15. Composition selon la revendication 14, caractérisée en ce que le composé hydrophile cosmétiquement ou dermatologiquement actif est choisi parmi les alcools à chaîne courte et les polyols.

16. Composition selon la revendication 14 ou 15, caractérisée en ce que le composé hydrophile cosmétiquement ou dermatologiquement actif est encapsulé dans un vésicule lipidique ionique et/ou non ionique ou dans une nanoparticule, nanosphère, nanoéponge ou nanocapsule.

17. Composition selon l'une quelconque des revendications précédentes, caractérisée en ce que les globules huileux contiennent au moins une substance grasse ou lipophile ayant une activité pour le soin de la peau.

18. Composition selon la revendication 17, caractérisée en ce que la substance grasse ou lipophile est choisie parmi les agents antioxydants, anti-radicaux libres, hydratants, mélanorégulateurs, accélérateurs de bronzage, dépigmentants, de coloration de la peau, liporégulateurs, amincissants, anti-acnéiques, antiséborrhéiques, anti-vieillissement, anti-rides, anti-UV, kératolytiques, anti-inflammatoires, rafraichissants, cicatrisants, protecteurs vasculaires, antibactériens, antifongiques, antiperspirants, déodorants, conditionneurs de la peau, immunomodulateurs, nourrissants et les huiles essentielles et les parfums.

19. Composition selon l'une quelconque des revendications 1 à 17, caractérisée en ce que les globules huileux contiennent au moins une substance grasse ou lipophile ayant une activité pour le soin du cheveu.

20. Composition selon la revendication 19, caractérisée en ce que la substance grasse ou lipophile est choisie parmi les agents mélanorégulateurs, liporégulateurs, antiséborrhéiques, anti-vieillissement, anti-UV, kératolytiques, antibactériens, antifongiques, antipelliculaires, anti-chute des cheveux, colorants capillaires, décolorants capillaires, réducteurs pour permanente, conditionneurs des cheveux et nourrissants.

21. Composition selon la revendication 18 ou 20, caractérisée en ce que la substance grasse ou lipophile est choisie parmi les composés suivants : D $\alpha$ tocophérol, DL $\alpha$ tocophérol, acétate de D $\alpha$ tocophérol, acétate DL $\alpha$ tocophérol, palmitate d'ascorbyle, glycérides de vitamine F, vitamines D, rétinol, esters de rétinol, $\beta$ carotène, D panthénol, farnésol, acétate de farnésyle, huiles riches en acides gras essentiels, acide n octanoyl-5 salicylique, acide salicylique, alkylesters d'$\alpha$-hydroxyacides, acide asiatique, acide madécassique, asiaticoside, extrait total de Centella Asiatica, acide $\beta$ glycyrrhétinique, $\alpha$ bisabolol, céramides, phytanetriol, sphingomyéline de lait, phospholipides d'origine marine riches en acides gras essentiels polyinsaturés, éthoxyquine, extrait de romarin, extrait de mélisse, quercetine, extrait de microalgues séchées, huile essentielle de bergamotte, octylmethoxycinnamate, butylméthoxydibenzoyl-méthane, octyl triazone, oxydes de fer jaune, brun, noir, rouge, oxydes de titane sous forme micrométrique, nanométrique ou enrobée, di tertiobutyl-3,5 hydroxy-4 benzylidène-3 camphre, 2-benzotriazol-2-yl-4-méthyl-6-[3-[1,3,3,3-tétraméthyl-1-[triméthylsilyl)oxy]disiloxanyl]-2-méthyl-propyl] phénol, huile perfluorée, huile de maïs hyperoxygénée.

22. Composition selon l'une quelconque des revendications précédentes, caractérisée en ce que le rapport huile/eau est au plus égal à 1.

23. Procédé de préparation d'une composition selon l'une quelconque des revendications précédentes, caractérisé en ce que dans une première étape on mélange sous agitation la phase huileuse comprenant le tensioactif lipophile, le tensioactif hydrophile, le lipide amphiphile ionique, le composé actif au plan cosmétique ou dermatologique et la phase aqueuse et dans une deuxième étape on soumet le mélange obtenu à une homogénéisation basée sur le principe de la cavitation.

24. Procédé selon la revendication 23, caractérisé en ce que l'homogénéisation basée sur le principe de la cavitation est obtenue soit à l'aide de hautes pressions comprises entre 200 et 1500 bars, soit à l'aide d'ultrasons, soit à l'aide d'homogénéisateurs équipés d'une tête rotor-stator.

25. Procédé selon la revendication 23 ou 24, caractérisée en ce que l'on introduit au moins un composé hydrophile cosmétiquement ou dermatologiquement actif à l'état libre dans la première étape.

26. Procédé selon l'une des revendications 23 à 25, caractérisé en ce que l'on mélange au moins un composé hydrophile cosmétiquement ou dermatologiquement actif à l'état encapsulé dans une troisième étape.

27. Emulsion du type huile dans eau formée de globules huileux pourvus chacun d'un enrobage cristal liquide lamellaire et dispersés dans une phase aqueuse, caractérisée en ce que chaque globule huileux est unitairement enrobé d'une couche monolamellaire ou oligolamellaire obtenue à partir d'au moins un agent tensioactif lipophile, d'au moins un agent tensioactif hydrophile et d'au moins un lipide amphiphile ionique conférant à l'émulsion un pH allant de 5,5 à 7,5 les globules huileux enrobés ayant un diamètre moyen inférieur à 500 nanomètres.

28. Emulsion selon la revendication 27, caractérisée en ce que le rapport huile/eau est au plus égal à 1.

## Claims

1. Cosmetic or dermatological composition consisting of an emulsion of oil-in-water type formed of oily globules which are each provided with a lamellar liquid crystal coating and are dispersed in an aqueous phase, characterized in that each oily globule containing at least one lipophilic compound which is cosmetically or dermatologically active is individually coated with a monolamellar or oligolamellar layer obtained from at least one lipophilic surface-active agent, from at least one hydrophilic surface-active agent and from at least one ionic amphiphilic lipid imparting to the emulsion a pH ranging from 5.5 to 7.5, the coated oily globules having a mean diameter of less than 500 nanometres.

2. Composition according to Claim 1, characterized in that the oily globules have a mean diameter of less than 200 nanometres.

3. Composition according to either of the preceding claims, characterized in that the lipophilic surface-active agent and the hydrophilic surface-active agent each contain at least one saturated fatty chain having more than approximately 12 carbon atoms, preferably between 16 and 22.

4. Composition according to any one of the preceding claims, characterized in that the lipophilic surface-active agent has an HLB (hydrophilic-lipophilic balance) between 2 and 5.

5. Composition according to Claim 4, characterized in that the lipophilic surface-active agent having an HLB between 2 and 5 is chosen from sucrose distearate, diglyceryl distearate, tetraglyceryl tristearate, decaglyceryl decastearate, diglyceryl monostearate, hexaglyceryl tristearate, decaglyceryl pentastearate, sorbitan monostearate, sorbitan tristearate, diethylene glycol monostearate, the ester of glycerol and palmitic and stearic acids, polyoxyethylenated monostearate 2 EO (containing 2 ethylene oxide units), glyceryl mono- and dibehenate and pentaerythritol tetrastearate.

6. Composition according to any one of the preceding claims, characterized in that the hydrophilic surface-active agent has an HLB between 8 and 12.

7. Composition according to Claim 6, characterized in that the hydrophilic surface-active agent having an HLB between 8 and 12 is chosen from polyoxyethylenated sorbitan monostearate 4 EO, polyoxyethylenated sorbitan tristearate 20 EO, polyoxyethylenated monostearate 8 EO, hexaglyceryl monostearate, polyoxyethylenated monostearate 10 EO, polyoxyethylenated distearate 12 EO and polyoxyethylenated methylglucose distearate 20 EO.

8. Composition according to any one of the preceding claims, characterized in that the ionic amphiphilic lipid is chosen from the group comprising anionic lipids which are neutralized, amphoteric lipids and alkylsulphonic derivatives.

9. Composition according to Claim 8, characterized in that the ionic amphiphilic lipid is chosen from alkali metal salts of dicetyl phosphate, alkali metal salts of dimyristyl phosphate, alkali metal salts of cholesteryl sulphate, alkali metal salts of cholesteryl phosphate, the mono- and disodium salts of acylglutamic acids, phospholipids and alkylsulphonic derivatives of formula:

$$R-\underset{\substack{|\\ SO_3M}}{CH}-CO-O-(CH_2CH_2O)_2-CH_3$$

in which R represents the radicals $C_{16}H_{33}$ and $C_{18}H_{37}$, taken as a mixture or separately, and M is an alkali metal.

10. Composition according to any one of the preceding claims, characterized in that the lipophilic surface-active agent, the hydrophilic surface-active agent and the ionic amphiphilic lipid are present in an amount between 2 and 6% by weight relative to the total weight of the composition and preferably between 3 and 4%.

11. Composition according to any one of the preceding claims, characterized in that the lipophilic surface-active agent, the hydrophilic surface-active agent and the ionic amphiphilic lipid preferably vary within the respective ranges of 35-55%, 25-40% and 15-35% by weight relative to their total weight.

12. Composition according to any one of the preceding claims, characterized in that the coated oily globules represent from 5 to 50% by weight relative to the total weight of the composition, preferably from 10 to 40%.

13. Composition according to any one of the preceding claims, characterized in that the weight ratio of the oily globules to the surfactants constituting the coating is between 2 and 13, preferably approximately equal to 7.

14. Composition according to any one of the preceding claims, characterized in that the aqueous phase additionally contains one or more free or encapsulated, cosmetically or dermatologically active hydrophilic compounds.

15. Composition according to Claim 14, characterized in that the cosmetically or dermatologically active hydrophilic compound is chosen from short-chain alcohols and polyols.

16. Composition according to Claim 14 or 15, characterized in that the cosmetically or dermatologically active hydrophilic compound is encapsulated in an ionic and/or nonionic lipid vesicle or in a nanoparticle, nanosphere, nanosponge or nanocapsule.

17. Composition according to any one of the preceding claims, characterized in that the oily globules contain at least one fatty or lipophilic substance having a skin-care activity.

18. Composition according to Claim 17, characterized in that the fatty or lipophilic substance is chosen from antioxidants, free radical scavengers, moisturizing agents, melanoregulators, tanning accelerators, depigmenting agents, skin-colouring agents, liporegulators, thinning agents, anti-acne agents, antiseborrhoeic agents, anti-ageing agents, anti-wrinkle agents, anti-UV agents, keratolytic agents, anti-inflammatory agents, refreshing agents, cicatrizing agents, vascular protectors, antibacterial agents, antifungal agents, antiperspirants, deodorants, skin conditioners, immunomodulators, nutrients and essential oils and perfumes.

19. Composition according to any one of Claims 1 to 17, characterized in that the oily globules contain at least one fatty or lipophilic substance having a hair-care activity.

20. Composition according to Claim 19, characterized in that the fatty or lipophilic substance is chosen from melanoregulators, liporegulators, anti-seborrhoeic agents, anti-ageing agents, anti-UV agents, keratolytic agents, antibacterial agents, antifungal agents, anti-dandruff agents, agents for combating hair loss, hair dyes, hair bleaches, reducing agents for permanent waves, hair conditioners and nutrients.

21. Composition according to Claim 18 or 20, characterized in that the fatty or lipophilic substance is chosen from the following compounds: D-$\alpha$-tocopherol, DL-$\alpha$-tocopherol, D-$\alpha$-tocopheryl acetate, DL-$\alpha$-tocopheryl acetate, ascorbyl palmitate, glycerides of vitamin F, D-vitamins, retinol, retinol esters, $\beta$-carotene, D-panthenol, farnesol, farnesyl acetate, oils rich in essential fatty acids, 5-n-octanoylsalicylic acid, salicylic acid, alkyl esters of $\alpha$-hydroxy acids, asiatic acid, madecassic acid, asiaticoside, whole extract of Centella asiatica, $\beta$-glycyrrhetinic acid, $\alpha$-bisabolol, ceramides, phytanetriol, sphingomyelin from milk, phospholipids of marine origin which are rich in polyunsaturated essential fatty acids, ethoxyquine, extract of romarin, extract of balm, quercetin, extract of dried microalgae, essential oil of bergamot, octyl methoxycinnamate, butylmethoxydibenzoylmethane, octyl triazone, yellow, brown, black and red iron oxides, titanium oxides which may be provided in micrometric or nanometric form or in coated form, 3,5-di-tert-butyl-4-hydroxybenzylidene-3-camphor, 2-benzotriazol-2-yl-4-methyl-6-[3-[1,3,3,3-tetramethyl-1-[trimethylsilyl)oxy]disiloxanyl]-2-methylpropyl]phenol, perfluoro oil and hyperoxygenated corn oil.

22. Composition according to any one of the preceding claims, characterized in that the oil/water ratio is at most equal to 1.

23. Process for the preparation of a composition according to any one of the preceding claims, characterized in that, in a first step, the oily phase comprising the lipophilic surfactant, the hydrophilic surfactant, the ionic amphiphilic lipid, the cosmetically or dermatologically active compound and the aqueous phase are mixed with stirring and, in a second step, the mixture obtained is subjected to a homogenization based on the principle of cavitation.

24. Process according to Claim 23, characterized in that the homogenization based on the principle of cavitation is obtained either using high pressures between 200 and 1500 bar or using ultrasound or using homogenizers equipped with a rotor-stator head.

25. Process according to Claim 23 or 24, characterized in that at least one cosmetically or dermatologically active hydrophilic compound in the free state is introduced in the first step.

26. Process according to one of Claims 23 to 25, characterized in that at least one cosmetically or dermatologically active hydrophilic compound in the encapsulated state is mixed in a third step.

27. Emulsion of the oil-in-water type formed of oily globules which are each provided with a lamellar liquid crystal coating and are dispersed in an aqueous phase, characterized in that each oily globule is individually coated with a monolamellar or oligolamellar layer obtained from at least one lipophilic surface-active agent, from at least one hydrophilic surface-active agent and from at least one ionic amphiphilic lipid imparting to the emulsion a pH ranging from 5.5 to 7.5, the coated oily globules having a mean diameter of less than 500 nanometres.

28. Emulsion according to Claim 27, characterized in that the oil/water ratio is at most equal to 1.

## Patentansprüche

1. Kosmetische oder dermatologische Zusammensetzung, die aus einer Öl-in-Wasser-Emulsion besteht, die aus in einer wäßrigen Phase dispergierten Ölkügelchen gebildet wird, welche jeweils mit einer lamellaren flüssigkristallinen Umhüllung versehen sind, dadurch gekennzeichnet, daß jedes Ölkügelchen, das mindestens eine kosmetisch oder dermatologisch wirksame lipophile Verbindung enthält, einheitlich mit einer monolamellaren oder oligolamellaren Schicht umhüllt ist, die aus mindestens einem lipophilen grenzflächenaktiven Stoff, mindestens einem hydrophilen grenzflächenaktiven Stoff und mindestens einem ionischen amphiphilen Lipid erhalten ist und die der Emulsion einen pH-Wert im Bereich von 5,5 bis 7,5 verleiht, wobei die umhüllten Öltröpfchen einen mittleren Durchmesser unter 500 Nanometern aufweisen.

2. Zusammensetzung nach Anspruch 1, dadurch gekennzeichnet, daß die Ölkügelchen einen mittleren Durchmesser unter 200 nm aufweisen.

3. Zusammensetzung nach einem der vorhergehenden Ansprüche, dadurch gekennzeichnet, daß der lipophile grenzflächenaktive Stoff und der hydrophile grenzflächenaktive Stoff jeweils mindestens eine gesättigte Fettkette mit mehr als etwa 12 Kohlenstoffatomen und vorzugsweise im Bereich von 16 bis 22 Kohlenstoffatomen enthalten.

4. Zusammensetzung nach einem der vorhergehenden Ansprüche, dadurch gekennzeichnet, daß der lipophile grenzflächenaktive Stoff einen HLB(hydrophilic lipophilic balance)-Wert im Bereich von 2 bis 5 aufweist.

5. Zusammensetzung nach Anspruch 4, dadurch gekennzeichnet, daß der lipophile grenzflächenaktive Stoff einen HLB-Wert im Bereich von 2 bis 5 aufweist und ausgewählt ist unter Saccharosedistearat, Diglyceryldistearat, Tetraglyceryltristearat, Decaglyceryldecastearat, Diglycerinmonostearat, Hexaglyceryltristearat, Decaglycerylpentastearat, Sorbitanmonostearat, Sorbitantristearat, Diethylenglykolmonostearat, dem Ester von Glycerin und Palmitin- und Stearinsäure, dem mit 2 mol EO polyethoxylierten Monostearat (das 2 Ethylenoxideinheiten aufweist), Glycerylmono- und -dibehenat sowie Pentaerythrittetrastearat.

6. Zusammensetzung nach einem der vorhergehenden Ansprüche, dadurch gekennzeichnet, daß der hydrophile grenzflächenaktive Stoff einen HLB-Wert im Bereich von 8 bis 12 aufweist.

7. Zusammensetzung nach Anspruch 6, dadurch gekennzeichnet, daß der hydrophile grenzflächenaktive Stoff einen HLB-Wert im Bereich von 8 bis 12 aufweist und ausgewählt ist unter mit 4 mol EO polyethoxyliertem Sorbitanmonostearat, mit 20 mol EO polyethoxyliertem Sorbitantristearat, Polyethylenglykolmonostearat mit 8 EO- Einheiten, Hexaglycerylmonostearat, Polyethylenglykolmonostearat mit 20 EO-Einheiten, Polyethylenglykoldistearat mit 12 EO- Einheiten und mit 20 EO polyethoxyliertem Methylglucosedistearat.

8. Zusammensetzung nach einem der vorhergehenden Ansprüche, dadurch gekennzeichnet, daß das amphiphile ionische Lipid ausgewählt ist unter den neutralisierten anionischen Lipiden, den amphoteren Lipiden und den Alkylsulfonsäurederivaten.

9. Zusammensetzung nach Anspruch 8, dadurch gekennzeichnet, daß das amphiphile ionische Lipid ausgewählt ist unter den Alkalisalzen von Dicetylphosphat, den Alkalisalzen von Dimyristylphosphat, den Alkalisalzen von Cholesterinsulfat, den Alkalisalzen von Cholesterinphosphat, den Mono- und Dialkalisalzen von Acylglutaminsäuren, Phospholipiden und Alkylsulfonsäurederivaten der Formel:

$R-CH-CO-O-(CH_2CH_2O)_2-CH_3$
|
$SO_3M$

worin bedeuten:

R $C_{16}H_{33}$ und/oder $C_{18}H_{37}$ und
M ein Alkalimetall.

**10.** Zusammensetzung nach einem der vorhergehenden Ansprüche, dadurch gekennzeichnet, daß der lipophile grenzflächenaktive Stoff, der hydrophile grenzflächenaktive Stoff und das amphiphile ionische Lipid in einem Mengenanteil im Bereich von 2 bis 6 Gew.-% und vorzugsweise von 3 bis 4 Gew.-%, bezogen auf das Gesamtgewicht der Zusammensetzung, vorliegen.

**11.** Zusammensetzung nach einem der vorhergehenden Ansprüche, dadurch gekennzeichnet, daß der lipophile grenzflächenaktive Stoff, der hydrophile grenzflächenaktive Stoff und das amphiphile ionische Lipid vorzugsweise in Anteilen von 35 bis 55 Gew.-%, 25 bis 40 Gew.-% bzw. 15 bis 35 Gew.-%, bezogen auf ihr Gesamtgewicht, vorliegen.

**12.** Zusammensetzung nach einem der vorhergehenden Ansprüche, dadurch gekennzeichnet, daß die umhüllten Ölkügelchen 5 bis 50 Gew.-%, bezogen auf das Gesamtgewicht der Zusammensetzung und vorzugsweise 10 bis 40 Gew.-% ausmachen.

**13.** Zusammensetzung nach einem der vorhergehenden Ansprüche, dadurch gekennzeichnet, daß das Gewichtsverhältnis von Öltröpfchen zu grenzflächenaktiven Stoffen, die die Umhüllung bilden, im Bereich von 2 bis 13 und vorzugsweise bei etwa 7 liegt.

**14.** Zusammensetzung nach einem der vorhergehenden Ansprüche, dadurch gekennzeichnet, daß die wäßrige Phase ferner einen oder mehrere hydrophile kosmetisch oder dermatologisch wirksame Verbindungen enthält, die frei oder eingekapselt vorliegen.

**15.** Zusammensetzung nach Anspruch 14, dadurch gekennzeichnet, daß die hydrophile kosmetisch oder dermatologisch wirksame Verbindung unter den kurzkettigen Alkoholen und den Polyolen ausgewählt ist.

**16.** Zusammensetzung nach Anspruch 14 oder 15, dadurch gekennzeichnet, daß die hydrophile kosmetisch oder dermatologisch wirksame Verbindung in ionischen und/oder nichtionischen Lipidvesikeln oder in Nanopartikeln, in Nanokugeln, Nanoschwämmen oder Nanokapseln eingekapselt ist.

**17.** Zusammensetzung nach einem der vorhergehenden Ansprüche, dadurch gekennzeichnet, daß die Öltröpfchen mindestens eine Fettsubstanz oder lipophile Substanz enthalten, die zur Pflege der Haut wirksam ist.

**18.** Zusammensetzung nach Anspruch 17, dadurch gekennzeichnet, daß die Fettsubstanz oder lipophile Substanz ausgewählt ist unter Antioxidantien, Mitteln gegen freie Radikale, Hydratisierungsmitteln, Melaninbildungsregulatoren, Bräunungsbeschleunigern, depigmentierenden Mitteln, Mitteln zur Färbung der Haut, Lipidregulatoren, schlankermachenden Mitteln, Mitteln gegen Akne, Mitteln gegen Seborrhoe, Mitteln gegen Alterung, Mitteln gegen Falten, Mitteln gegen UV-Strahlung, Keratolytika, entzündungshemmenden Mitteln, erfrischenden Mitteln, wundheilenden Mitteln, vaskulären Schutzmitteln, antibakteriellen Mitteln, Antimykotika, Antitranspirantien, Deodorantien, Hautkonditionierern, Immunmodulatoren, nährenden Mitteln und etherischen Ölen und Parfums.

**19.** Zusammensetzung nach einem der Ansprüche 1 bis 17, dadurch gekennzeichnet, daß die Öltröpfchen mindestens eine Fettsubstanz oder lipophile Substanz mit haarpflegender Wirkung enthalten.

**20.** Verbindung nach Anspruch 19, dadurch gekennzeichnet, daß die Fettsubstanz oder lipophile Substanz ausgewählt ist unter Melaninbildungsregulatoren, Lipidregulatoren, Mitteln gegen Seborrhoe, Mitteln gegen Alterung, Mitteln gegen UV-Strahlung, Keratolytika, antibakteriellen Mitteln, Antimykotika, Mitteln gegen Schuppen, Mitteln gegen

Haarausfall, Haarfärbemitteln, Haarentfärbemitteln, reduzierenden Mitteln für Dauerwellen, Haarkonditionierern und nährenden Mitteln.

**21.** Zusammensetzung nach Anspruch 18 oder 20, dadurch gekennzeichnet, daß die Fettsubstanz oder lipophile Substanz unter den folgenden Verbindungen ausgewählt ist: D-α-Tocopherol, D,L-α-Tocopherol, Acetat von DL-α-Tocopherol, Acetat von D,L-α-Tocopherol, Ascorbylpalmitat, Glyceriden von Vitamin F, Vitaminen D, Retinol, Retinolestern, β-Carotin, D-Panthenol, Farnesol, Farnesylacetat, Ölen, die reich an essentiellen Fettsäuren sind, 5-(n-Octanoyl)salicylsäure, Salicylsäure, Alkylestern von α-Hydroxysäuren, Asiatsäure, madegassischer Säure, Asiaticosid, Gesamtextrakt von Centella Asiatica, β-Glycyrrhetinsäure, α-Bisabolol, Ceramiden, Phytantriol, Sphingomyelin aus Milch, Phospholipiden maritimen Ursprungs, die reich an mehrfach ungesättigten essentiellen Fettsäuren sind, Ethoxyquin, Rosmarinextrakt, Melissenextrakt, Quercetin, Extrakt aus getrockneten Mikroalgen, etherisches Bergamotteöl, Octylmethoxycinnamat, Butylmethoxydibenzoylmethan, Octyltriazon, gelbem, braunem, schwarzem und rotem Eisenoxid, Titanoxiden, deren Größe in Mikrometer- und Nanometerbereich liegt oder die umhüllt vorliegen, Di-*tert*.-butyl-3,5-hydroxy-4-benzyliden-3-campher, 2-Benzotriazol-2-yl-4-methyl-6-[3-(1,3,3,3-tetramethyl-1-[trimethylsilyloxy]-disiloxanyl)-2-methyl-propyl]-phenol, perfluoriertem Öl und peroxidiertes Maisöl.

**22.** Zusammensetzung nach einem der vorhergehenden Ansprüche, dadurch gekennzeichnet, daß das Verhältnis Öl zu Wasser höchstens 1 beträgt.

**23.** Verfahren zur Herstellung einer Zusammensetzung nach einem der vorhergehenden Ansprüche, dadurch gekennzeichnet, daß in einem ersten Schritt die Ölphase, die den lipophilen grenzflächenaktiven Stoff, den hydrophilen grenzflächenaktiven Stoff, das amphiphile ionische Lipid und die kosmetisch oder dermatologisch wirksame Verbindung enthält, unter Rühren mit der wäßrigen Phase vermischt wird, und in einem zweiten Schritt das erhaltene Gemisch einer Homogenisierung unterzogen wird, die auf dem Prinzip der Kavitation beruht.

**24.** Verfahren nach Anspruch 23, dadurch gekennzeichnet, daß die Homogenisierung, die auf dem Prinzip der Kavitation beruht, mit hohen Drücken im Bereich von 200 bis 1500 bar oder mit Ultraschall oder mit einem Homogenisator, der mit einem Rotor-Stator-Kopf ausgestattet ist, erzielt wird.

**25.** Verfahren nach Anspruch 23 oder 24, dadurch gekennzeichnet, daß im ersten Schritt mindestens eine hydrophile kosmetisch oder dermatologisch wirksame Verbindung in freiem Zustand zugesetzt wird.

**26.** Verfahren nach einem der Ansprüche 23 bis 25, dadurch gekennzeichnet, daß in einem dritten Schritt mindestens eine hydrophile kosmetisch oder dermatologisch wirksame Verbindung in eingekapseltem Zustand vermischt wird.

**27.** Öl-in-Wasser-Emulsion, die aus in einer wäßrigen Phase dispergierten Öltröpfchen gebildet wird, die jedes mit einer lamellaren flüssigkristallinen Umhüllung versehen sind, dadurch gekennzeichnet, daß jedes Öltröpfchen gleichmäßig mit einer monolamellaren oder oligolamellaren Schicht umhüllt ist, die aus mindestens einem lipophilen grenzflächenaktiven Stoff, mindestens einem hydrophilen grenzflächenaktiven Stoff und mindestens einem amphiphilen ionischen Lipid erhalten ist und die der Emulsion einen pH-Wert im Bereich von 5,5 bis 7,5 verleiht, wobei die umhüllten Öltröpfchen einen mittleren Durchmesser von unter 500 Nanometern aufweisen.

**28.** Emulsion nach Anspruch 27, dadurch gekennzeichnet, daß das Verhältnis von Öl zu Wasser höchstens 1 beträgt.